# EUROPÄISCHE PATENTSCHRIFT

(11) **EP 2 015 703 B1**
(45) Veröffentlichungstag und Bekanntmachung des Hinweises auf die Patenterteilung: **20.07.2011**
(21) Anmeldenummer: 07728700.1
(22) Anmeldetag: 02.05.2007
(51) Int. Cl.: A61F 2/00, A61F 2/30, A61F 2/36, A61F 2/46

(54) **VORRICHTUNG ZUR MONTAGE VON KUGELKÖPFEN UND ADAPTERHÜLSEN ALS INTEGRIERTER BESTANDTEIL DER VERPACKUNG**
DEVICE FOR ASSEMBLY OF BALL HEADS AND ADAPTER SLEEVES AS INTEGRATED COMPONENT PART OF THE PACKAGE
DISPOSITIF DE MONTAGE DE ROTULES ET DE MANCHONS D'ADAPTATEUR SOUS FORME DE CONSTITUANT INTEGRE DE L'EMBALLAGE

(30) Priorität: 02.05.2006 DE 102006020616; 20.06.2006 DE 202006009757 U; 20.06.2006 DE 102006028720
(43) Veröffentlichungstag der Anmeldung: 21.01.2009
(73) Patentinhaber: CeramTec GmbH, 73207 Plochingen (DE)
(72) Erfinder: DIETRICH, Martin, 23942 Pötenitz (DE); MERKERT, Patricie, 73230 Kirchheim u. Teck (DE); PREUSS, Roman, 73230 Kirchheim unter Teck (DE); SILBERER, Paul, 68753 Waghäusel (DE); WECKER, Heinrich, 90542 Eckental (DE)
(74) Vertreter: Uppena, Franz
(86) Internationale Anmeldenummer: PCT/EP2007/054246
(87) Internationale Veröffentlichungsnummer: WO 2007/125125

(56) Entgegenhaltungen:
- EP-A- 0 133 393
- EP-A- 0 373 078
- EP-A- 0 523 895
- EP-A- 0 547 354
- EP-A- 1 405 617
- FR-A- 2 656 792
- US-A- 4 921 500
- US-A1- 2004 054 373

## Beschreibung

Die Erfindung betrifft eine Verpackung zur Aufnahme einer Adapterhülse für eine Hüftendoprothese, wobei die Hüftendoprothese einen Kugelkopf umfasst, der mit einer definierten Aufsteckkraft auf die Adapterhülse aufgesteckt sein muss.

Im Markt existieren verschiedene modulare Systeme für Hüftendoprothesen, bei denen der Kugelkopf auf einer Adapterhülse und diese wiederum auf dem Prothesenkonus sitzt. In der Regel handelt es sich dabei um Bauteile, die über konische Klemmverbindungen miteinander verbunden sind. In der Regel werden weiterhin alle Teile getrennt und nicht vormontiert ausgeliefert, um Veränderungen der Steckverbindungen infolge äußerer Einflüsse beim Transport zu vermeiden.

Dabei treten drei Probleme auf.
1 Für den Anwender, der die Montage der Bauteile vornimmt, muss deutlich sein, in welcher Reihenfolge die Montage durchzuführen ist.
2 Für den Anwender muss gerade im Fall konischer Steckverbindungen klar sein, mit welcher initialen Kraft die Bauteile zu fügen sind.
3 Die Steckverbindung Kugelkopf-Adapterhülse muss sauber sein. Es dürfen keine Schmutzteilchen auf der Kontaktfläche vorhanden sein.

FR-A- 2 656 792 offenbart eine Verpackung zur Aufnahme eines Kugelkopfes einer Hüftendoprothese, in welcher eine Vorrichtung zur Montage des Kugelkopfes auf einen Prothesenkonus integriert ist.

Die vorliegende Erfindung bietet für alle drei Probleme eine Lösung an:

Erfindungsgemäß ist in die Verpackung eine Vorrichtung zur Montage des Kugelkopfes auf die Adapterhülse integriert und umfasst diese Vorrichtung Anzeigeelemente, die beim Aufdrücken des Kugelkopfes auf die Adapterhülse das Erreichen der definierten Aufsteckkraft anzeigen. Hierdurch ist die Montage vereinfacht und der Anwender erkennt, ob er den Kugelkopf mit der notwendigen Aufsteckkraft auf die Adapterhülse aufgesteckt hat.

In einer erfindungsgemäßen Ausführungsform umfasst die Vorrichtung zur Montage eine bis zu einem Anschlag eindrückbare Ausbuchtung der Verpackung, auf welche die Adapterhülse aufgesetzt ist und, beim Aufdrücken des Kugelkopfes auf die Adapterhülse, der Anschlag das Erreichen der notwendigen Aufsteckkraft anzeigt.

Das Material und/oder die Ausgestaltung der eindrückbaren Ausbuchtung muss so gewählt sein, dass die Ausbuchtung den Anschlag erst berührt, wenn die notwendige Aufsteckkraft erreicht ist. Der Anschlag kann auch der Boden der Verpackung sein.

Bevorzugt ist die Ausbuchtung im Bereich des stirnseitigen Endes der Adapterhülse einknickbar ausgebildet.

In einer anderen Ausführungsform ist die Vorrichtung zur Montage des Kugelkopfes auf die Adapterhülse als nachgiebige, umschlagfähige Struktur ausgebildet und zeigt beim Aufdrücken des Kugelkopfes auf die Adapterhülse das Umschlagen der Struktur das Erreichen der notwendigen Aufsteckkraft an.

Bevorzugt ist die Struktur so ausgebildet, dass sie beim Umschlagen einen Ton, wie z.B. ein Klicken erzeugt.

In vorteilhafter Ausgestaltung ist zwischen der Adapterhülse und der Ausbuchtung oder der Verpackung eine Anti-Verpackungsabrieb-Scheibe angeordnet, so dass die Adapterhülse die Ausbuchtung und/oder die Verpackung nicht berührt. Hierdurch tritt kein Abrieb an der Verpackung auf, der sich an der Adapterhülse oder dem Kugelkopf absetzen und zu Problemen bei der Implantation führen könnte.

In einer erfindungsgemäßen Ausführungsform ist die Anti-Verpackungsabrieb-Scheibe als Luftpolster ausgeführt, welches platzt, wenn die Aufsteckkraft des Kugelkopfes auf die Adapterhülse groß genug ist. Das Platzen zeigt dem Anwender an, dass die Aufsteckkraft groß genug ist.

In einer anderen Ausführungsform der Erfindung ist die Anti-Verpackungsabrieb-Scheibe als räumliches, durch Krafteinwirkung verformbares Gebilde ausgeführt, wobei das Gebilde voneinander beabstandete Mittel umfasst, die sich erst berühren, wenn die Aufsteckkraft des Kugelkopfes auf die Adapterhülse groß genug ist.

Vorteilhafterweise sind die Mittel Plättchen, die durch einen Abstandshalter voneinander getrennt sind.

In einer Ausführungsform der Erfindung wird durch die Materialien oder Beschichtung der Mittel oder der Plättchen bei Berührung eine chemische Reaktion mit Farbumschlag ausgelöst. Hierdurch erkennt der Anwender, wann die Aufsteckkraft groß genug ist.

In einer alternativen erfindungsgemäßen Ausführungsform ist zwischen der Adapterhülse und der Verpackung ggf. zusätzlich zur Anti-Verpackungsabrieb-Scheibe ein Piezoelement angeordnet, welches die Aufsteckkraft misst und anzeigt bzw. die Anzeige initiiert, wobei das Anzeigeelement zum Beispiel eine Lampe oder ein Schallgeber sein kann.

In einer anderen Ausführungsform der Erfindung ist die Ausbuchtung als konischer Zapfen ausgebildet auf dem eine Anti-Verpackungsabrieb-Scheibe bei Druckbeaufschlagung in Längsrichtung des Zapfens verschiebbar angeordnet ist und die Adapterhülse den Zapfen umgreifend auf der Anti-Verpackungsabrieb-Scheibe aufsitzt und beim Aufdrücken des Kugelkopfes auf die Adapterhülse die Anti-Verpackungsabrieb-Scheibe verschiebt.

Vorteilhafterweise ist die Stelle, bis zu der bei Druckbeaufschlagung der Adapterhülse durch den Kugelkopf, die Anti-Verpackungsabrieb-Scheibe verschoben werden muss, gekennzeichnet oder ist ein Anschlag, wie zum Beispiel der Boden der Verpackung.

Vorteilhaft ist in der Verpackung zugleich auch ein Kugelkopf herausnehmbar positioniert, wobei der Kugelkopf einzeln entnehmbar ist.

Die Erfindung zeichnet sich also dadurch aus, dass der Kugelkopf und die Adapterhülse in einer Verpackung so positioniert sind, dass der Kugelkopf einzeln entnommen werden kann. Die Adapterhülse verbleibt in der Verpackung und der Kugelkopf wird dann direkt auf die noch in der Verpackung befindliche Adapterhülse gesteckt. Des Weiteren ist in die Verpackung eine Vorrichtung integriert, die beim Aufdrücken des Kugelkopfes auf die Adapterhülse eine Rückmeldung an den Anwender liefert, wenn die notwendige Aufsteckkraft erreicht ist. Die Rückmeldung kann dabei z.B. taktil oder durch visuell bzw. Audio-Signale erfolgen.

Nachfolgend wird die Erfindung anhand verschiedener Figuren weiter erläutert.

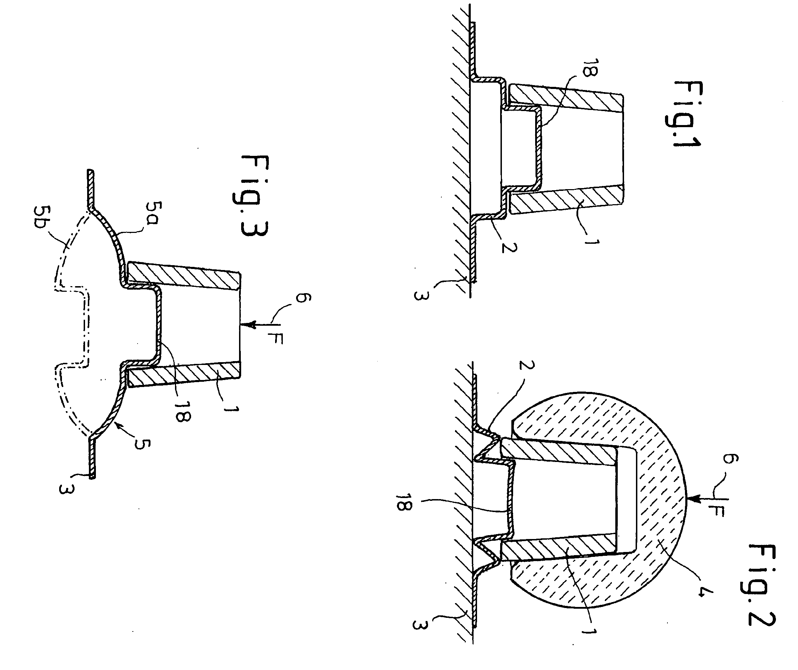

Figur 1 zeigt im Querschnitt eine Adapterhülse 1, die erhöht auf einer zylindrischen Ausbuchtung 2 einer Verpackung 3 sitzt. Dies ist der Auslieferungszustand der Verpackung 3. Nicht gezeigt ist, dass zusätzlich zur Adapterhülse 1 in der Verpackung 3 ' auch ein Kugelkopf herausnehmbar in irgendeiner Weise angeordnet ist. Damit sich die Adapterhülse 1 auf der Ausbuchtung 2 nicht verschiebt, ist auf der Ausbuchtung 2 ein Zylinder 18 angeordnet, dessen Durchmesser dem Innendurchmesser der Adapterhülse 1 am stirnseitigen Ende entspricht.
Figur 2 zeigt die Verpackung 3 gemäß Figur 1 kurz nach der Anwendung. Der Kugelkopf 4 ist auf die Adapterhülse 1 aufgesetzt. Mit ansteigendem Druck des Kugelkopfes 4 auf die Adapterhülse 1 gibt die zylindrische Ausbuchtung 2 nach, bis die Unterlage der Verpackung 3 erreicht wird. Die Unterlage wirkt sozusagen als mechanischer Anschlag und signalisiert, dass eine ausreichende Kraft aufgebracht wurde (taktile Rückmeldung). Das Material oder die Ausgestaltung der zylindrischen Ausbuchtung 2 muss so gewählt werden, dass sie die Unterlage der Verpackung 3 erst berührt, wenn der Kugelkopf 4 mit ausreichendem Druck auf die Adapterhülse 1 gedrückt wird.
Figur 3 zeigt eine Ausführungsform, bei der die Ausbuchtung der Verpackung 3 als nachgiebige, umschlagfähige Struktur 5 ausgeführt ist, so dass bei Erreichen der notwendigen Kraft die Struktur 5 umschlägt und ein Klicken zu hören ist (taktile und Audio-Rückmeldung). Mit dem Bezugszeichen 5a ist die Struktur im nicht umgeschlagenen Zustand, d.h. im Ausgangszustand gekennzeichnet. Die gestrichelte Linie 5b zeigt die Struktur im umgeschlagenen Zustand, d.h. dass die Kraftbeaufschlagung ausgereicht hat.

Die Kraftbeaufschlagung durch den Kugelkopf ist in dieser Figur und auch den nachfolgenden Figuren durch den Pfeil 6 gekennzeichnet.

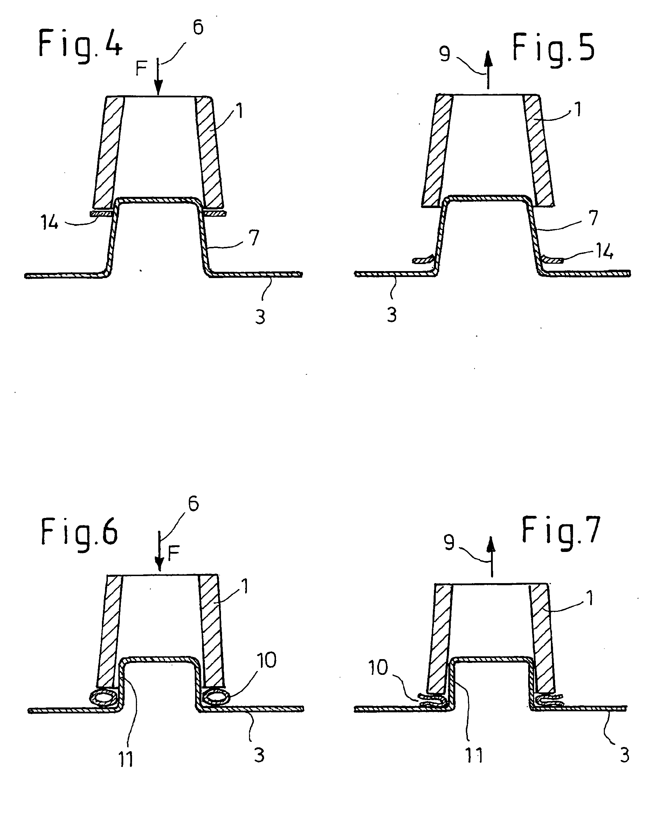

Figur 4 zeigt eine Ausbuchtung der Verpackung 3, die als konischer Zapfen 7 ausgeführt ist, wobei die Adapterhülse 1 auf diesen Zapfen 7 aufgesteckt wird. Unter der Adapterhülse 1 auf dem Zapfen 7 der Ausbuchtung sitzt eine Anti-Verpackungsabrieb-Scheibe 14, welche verhindern soll, dass sich Adapterhülse 1 und Verpackung 3 berühren. Hierdurch tritt kein Abrieb an der Verpackung 3 auf, der sich an der Adapterhülse 1 oder dem Kugelkopf absetzen und zu Problemen bei der Implantation führen könnte.

Wenn die Anti-Verpackungsabrieb-Scheibe 14, nach dem Aufdrücken des Kugelkopfes auf die Adapterhülse 1, auf dem konischen Zapfen 7, definiert verschoben verbleibt, dann war die Aufsteckkraft ausreichend. Die Stelle bis zu der die Anti-Verpackungsabrieb-Scheibe 14 verschoben werden muss, damit die Aufsteckkraft ausreicht, wird vorteilhaft gekennzeichnet oder ist der Boden der Verpackung 3.

Figur 5 zeigt die Verpackung gemäß Figur 4 im verschobenen Zustand der Anti-Verpackungsabrieb-Scheibe 14. Mit dem Pfeil 9 ist das Abheben des Kugelkopfes, der nun fest mit der Adapterhülse 1 verbunden ist, vom Zapfen 7 bezeichnet. Der Kugelkopf ist hier nicht gezeigt.

Figur 6 zeigt eine Ausführungsform, bei der die Anti-Verpackungsabrieb-Scheibe als Luftpolster 10 ausgeführt ist, welches platzt, wenn der Kugelkopf ausreichend stark auf die Adapterhülse 1 gedrückt wird. Dies zeigt Figur 7. Die Ausbuchtung 11 ist in dieser Ausführungsform bevorzugt zylindrisch ausgebildet und entspricht dem Zylinder 18 von Figur 1.

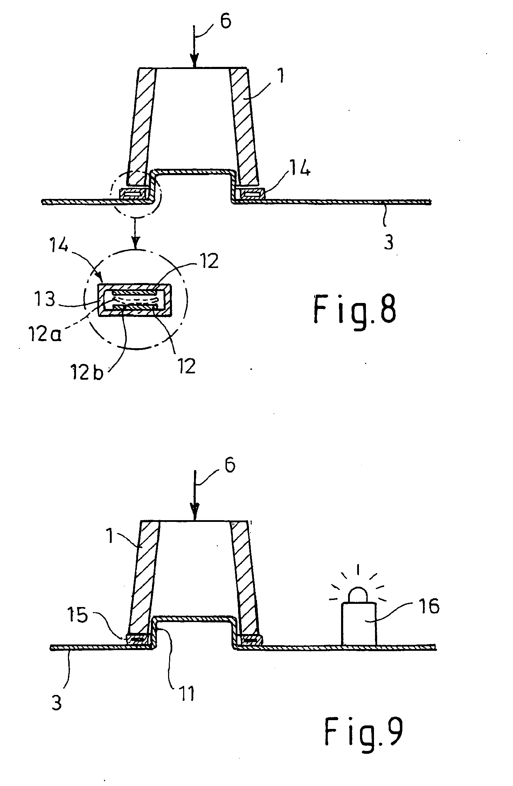

Figur 8 zeigt eine Anti-Verpackungsabrieb-Scheibe 14, die aus zwei Plättchen 12 besteht, welche durch einen Abstandshalter 13 getrennt sind. Wird durch den Kugelkopf genügend Druck auf die Adapterhülse 1 ausgeübt, wird mindestens eines der Plättchen 12 in der Anti-Verpackungsabrieb-Scheibe 14 soweit verformt, dass es das andere Plättchen 12 berührt. Dies könnte auf verschiedene Arten angezeigt werden. Bevorzugt könnte bedingt durch die Materialien oder Beschichtungen der Plättchen 12 eine chemische Reaktion mit Farbumschlag ausgelöst werden. Mit der gestrichelten Linie 12 a ist das durch Krafteinwirkung verbogene Plättchen bezeichnet, welches das andere Plättchen 12 b berührt.

Figur 9 zeigt eine Ausführungsform bei der elektronisch/elektrisch mit z. B. Piezoelementen 15 und Rückmeldung an einen Signalgeber 16 ein ausreichender Druck des Kugelkopfes auf die Adapterhülse 1 oder auf die Anti-Verpackungsabrieb-Scheibe gemessen und angezeigt wird. Der Signalgeber 16 könnte z. B. eine Lampe oder auch ein Schallgeber sein.

## Patentansprüche

1. Verpackung (3) zur Aufnahme einer Adapterhülse (1) für eine Hüftendoprothese, wobei die Hüftendoprothese einen Kugelkopf (4) umfasst, der mit einer definierten Aufsteckkraft auf die Adapterhülse (1) aufgesteckt sein muss,
wobei in die Verpackung (3) eine Vorrichtung zur Montage des Kugelkopfes (4) auf die Adapterhülse (1) integriert ist und diese Vorrichtung Anzeigeelemente umfasst, die beim Aufdrücken des Kugelkopfes (4) auf die Adapterhülse (1) das Erreichen der definierten Aufsteckkraft anzeigen.

2. Verpackung nach Anspruch 1,
**dadurch gekennzeichnet, dass** die Vorrichtung zur Montage eine bis zu einem Anschlag eindrückbare Ausbuchtung (2) der Verpackung (3) umfasst, auf welche die Adapterhülse (1) aufgesetzt ist und beim Aufdrücken des Kugelkopfes (4) auf die Adapterhülse (1) der Anschlag das Erreichen der notwendigen Aufsteckkraft anzeigt.

3. Verpackung nach Anspruch 2,
**dadurch gekennzeichnet, dass** das Material und/oder die Ausgestaltung der eindrückbaren Ausbuchtung (2) so gewählt ist, dass die Ausbuchtung (2) den Anschlag erst berührt, wenn die notwendige Aufsteckkraft erreicht ist.

4. Verpackung nach Anspruch 2 oder 3,
**dadurch gekennzeichnet, dass** die Ausbuchtung (2) im Bereich des stirnseitigen Endes der Adapterhülse (1) einknickbar ausgebildet ist.

5. Verpackung nach Anspruch 1 oder 2,
**dadurch gekennzeichnet, dass** die Vorrichtung zur Montage des Kugelkopfes (4) auf die Adapterhülse (1) als nachgiebige, umschlagfähige Struktur (5) ausgebildet ist und beim Aufdrücken des Kugelkopfes (4) auf die Adapterhülse (1), das Umschlagen der Struktur (5) das Erreichen der notwendigen Aufsteckkraft anzeigt.

6. Verpackung nach Anspruch 5,
**dadurch gekennzeichnet, dass** die Struktur (5) so ausgebildet ist, dass sie beim Umschlagen einen Ton, wie z.B. ein Klicken erzeugt.

7. Verpackung nach einem der vorhergehenden Ansprüche,
**dadurch gekennzeichnet, dass** zwischen der Adapterhülse (1) und der Ausbuchtung (2) oder der Verpackung (3) eine Anti-Verpackungsabrieb-Scheibe (14) angeordnet ist, so dass die Adapterhülse (1) die Ausbuchtung (2) und/oder die Verpackung (3) nicht berührt.

8. Verpackung nach Anspruch 7,
**dadurch gekennzeichnet, dass** die Anti-Verpackungsabrieb-Scheibe (14) als Luftpolster (10) ausgeführt ist, welches platzt, wenn die Aufsteckkraft des Kugelkopfes (4) auf die Adapterhülse (1) groß genug ist.

9. Verpackung nach Anspruch 7 oder 8,
**dadurch gekennzeichnet, dass** die Anti-Verpackungsabrieb-Scheibe (14) als räumliches, durch Krafteinwirkung verformbares Gebilde ausgeführt ist, wobei das Gebilde voneinander beabstandete Mittel umfasst, die sich erst berühren, wenn die Aufsteckkraft des Kugelkopfes (4) auf die Adapterhülse (1) groß genug ist.

10. Verpackung nach Anspruch 9,
**dadurch gekennzeichnet, dass** die Mittel Plättchen (12) sind, die durch einen Abstandshalter (12) voneinander getrennt sind.

11. Verpackung nach Anspruch 8 oder 9,
**dadurch gekennzeichnet, dass** durch die Materialien oder Beschichtung der Mittel oder der Plättchen (12) bei Berührung eine chemische Reaktion mit Farbumschlag ausgelöst wird.

12. Verpackung nach einem der vorhergehenden Ansprüche,
**dadurch gekennzeichnet, dass** zwischen der Adapterhülse (1) und der Verpackung (3) ggf. zusätzlich zur Anti-Verpackungsabrieb-Scheibe (14) ein Piezoelement (15) angeordnet ist, welches die Aufsteckkraft misst und anzeigt, wobei das Anzeigeelement zum Beispiel eine Lampe (17) oder ein Schallgeber sein kann.

13. Verpackung nach einem der vorhergehenden Ansprüche,
**dadurch gekennzeichnet, dass** die Ausbuchtung als konischer Zapfen (7) ausgebildet ist auf dem eine Anti-Verpackungsabrieb-Scheibe (14) bei Druckbeaufschlagung in Längsrichtung des Zapfens (7) verschiebbar angeordnet ist und die Adapterhülse (1) den Zapfen (7) umgreifend auf der Anti-Verpackungsabrieb-Scheibe (14) aufsitzt und beim Aufdrücken des Kugelkopfes (4) auf die Adapterhülse (1) die Anti-Verpackungsabrieb-Scheibe (14) verschiebt.

14. Verpackung nach einem der vorhergehenden Ansprüche,
**dadurch gekennzeichnet, dass** die Stelle, bis zu der bei Druckbeaufschlagung der Adapterhülse (1) durch den Kugelkopf (4), die Anti-Verpackungsabrieb-Scheibe (14) verschoben werden muss, **gekennzeichnet** ist oder ein Anschlag, wie zum Beispiel der Boden der Verpackung ist.

15. Verpackung nach einem der vorhergehenden Ansprüche,
**dadurch gekennzeichnet, dass** in der Verpackung (3) zugleich auch ein Kugelkopf (4) herausnehmbar positioniert ist und der Kugelkopf einzeln entnehmbar ist.

## Claims

1. A package (3) for receiving an adapter sleeve (1) for a hip endoprosthesis, the hip endoprosthesis comprising a ball head (4) which has to be pushed onto the adapter sleeve (1) with a defined pushing-on force,
wherein a device for the mounting of the ball head (4) on the adapter sleeve (1) is integrated in the package (3) and this device comprises indicating elements which indicate that the defined pushing-on force has been reached when the ball head (4) is pressed onto the adapter sleeve (1).

2. A package according to Claim 1,
**characterised in that** the device for the mounting comprises a projection (2) of the package (3), which projection can be pressed in as far as a stop and on which the adapter sleeve (1) is placed, and the stop indicates that the necessary pushing-on force has been reached when the ball head (4) is pressed onto the adapter sleeve (1).

3. A package according to Claim 2,
**characterised in that** the material and/or the configuration of the projection (2) which can be pressed in is chosen so that the projection (2) touches the stop only when the necessary pushing-on force has been reached.

4. A package according to Claim 2 or 3,
**characterised in that** the projection (2) is designed so that it can buckle in the region of the front end of the adapter sleeve (1).

5. A package according to Claim 1 or 2,
**characterised in that** the device for the mounting of the ball head (4) on the adapter sleeve (1) is designed as a yielding, collapsible structure (5) and the collapsing of the structure (5) indicates that the necessary pushing-on force has been reached when the ball head (4) is pressed onto the adapter sleeve (1).

6. A package according to Claim 5,
**characterised in that** the structure (5) is designed so that it produces a sound, such as, for example, a click, when it collapses.

7. A package according to one of the preceding claims,
**characterised in that** a package anti-abrasion disc (14) is arranged between the adapter sleeve (1) and the projection (2) or the package (3), so that the adapter sleeve (1) does not touch the projection (2) and/or the package (3).

8. A package according to Claim 7,
**characterised in that** the package anti-abrasion disc (14) is designed as an air cushion (10) which bursts when the force with which the ball head (4) is pushed onto the adapter sleeve (1) is large enough.

9. A package according to Claim 7 or 8,
**characterised in that** the package anti-abrasion disc (14) is designed as a spatial structure deformable through the effect of force, the structure comprising means which are spaced from one another and touch only when the force with which the ball head (4) is pushed onto the adapter sleeve (1) is large enough.

10. A package according to Claim 9,
**characterised in that** the means are laminas (12) which are separated from one another by a spacer (12).

11. A package according to Claim 8 or 9,
**characterised in that** a chemical reaction with a change of colour is triggered by the materials or coating of the means or the laminas (12) when they touch.

12. A package according to one of the preceding claims,
**characterised in that** a piezoelectric element (15) which measures and indicates the pushing-on force is arranged between the adapter sleeve (1) and the package (3), in addition to the package anti-abrasion disc (14), as the case may be, it being possible for the indicating element to be, for example, a lamp (17) or a sound generator.

13. A package according to one of the preceding claims,
**characterised in that** the projection is designed as a conical peg (7) on which a package anti-abrasion disc (14) is arranged in a manner displaceable in the longitudinal direction of the peg (7) on pressure application, and the adapter sleeve (1) when encompassing the peg (7) sits on the package anti-abrasion disc (14) and displaces the package anti-abrasion disc (14) when the ball head (4) is pressed onto the adapter sleeve (1).

14. A package according to one of the preceding claims,
**characterised in that** the point up to which the package anti-abrasion disc (14) has to be displaced when pressure is applied to the adapter sleeve (1) by the ball head (4) is marked or is a stop, such as, for example, the bottom of the package.

15. A package according to one of the preceding claims,
**characterised in that** a ball head (4) is also removably positioned in the package (3) at the same time, and the ball head can be removed separately.

## Revendications

1. Emballage (3) de réception d'un manchon d'adaptateur (1) pour une endoprothèse de hanche, l'endoprothèse comprenant une tête sphérique (4) qui doit être rapportée avec une force d'emmanchement définie sur le manchon d'adaptateur (1),
un dispositif pour le montage de la tête sphérique (4) sur le manchon d'adaptateur (1) étant intégré dans l'emballage (3), et ledit dispositif comprenant des éléments de signalisation qui, lorsque la tête sphérique (4) est pressée sur le manchon d'adaptateur (1), indiquent que la force d'emmanchement définie est atteinte.

2. Emballage selon la revendication 1, **caractérisé par le fait que** le dispositif de montage comporte une saillie (2) de l'emballage (3) qui peut être enfoncée jusqu'à une butée et sur laquelle est posé le manchon d'adaptateur (1) et, lorsque la tête sphérique (4) est pressée sur le manchon d'adaptateur (1), la butée indique que la force d'emmanchement nécessaire est atteinte.

3. Emballage selon la revendication 2, **caractérisé par le fait que** la matière et/ou la forme de la saillie (2) sont choisies de façon telle que la saillie (2) n'entre en contact avec la butée que lorsque la force d'emmanchement nécessaire est atteinte.

4. Emballage selon la revendication 2 ou 3, **caractérisé par le fait que** la saillie (2) est réalisée de manière à pouvoir plier dans la région de l'extrémité frontale du manchon d'adaptateur (1).

5. Emballage selon la revendication 1 ou 2, **caractérisé par le fait que** le dispositif de montage de la tête sphérique (4) sur le manchon d'adaptateur (1) est réalisé sous la forme d'une structure (5) souple, pouvant être retournée, et, lors de l'emmanchement de la tête sphérique (4) sur le manchon d'adaptateur (1), le retournement de la structure (5) indique que la force d'emmanchement nécessaire est atteinte.

6. Emballage selon la revendication 5, **caractérisé par le fait que** la structure (5) est réalisée de manière telle que, lors du retournement, elle produise un son, par exemple un clic.

7. Emballage selon une des revendications précédentes, **caractérisé par le fait qu'**un disque anti-usure d'emballage (14) est placé entre le manchon d'adaptateur (1) et la saillie (2) ou l'emballage (3), de sorte que le manchon d'adaptateur (1) n'entre pas en contact avec la saillie (2) et/ou l'emballage (3).

8. Emballage selon la revendication 7, **caractérisé par le fait que** le disque anti-usure d'emballage (14) est réalisé sous forme de coussin d'air (10) qui éclate lorsque la force d'emmanchement de la tête sphérique (4) sur le manchon d'adaptateur (1) est suffisamment grande.

9. Emballage selon la revendication 7 ou 8, **caractérisé par le fait que** le disque anti-usure d'emballage (14) est réalisé sous forme de structure tridimensionnelle, déformable par l'action d'une force, ladite structure comprenant des moyens qui sont espacés l'un de l'autre et entrent en contact l'un avec l'autre uniquement lorsque la force d'emmanchement de la tête sphérique (4) sur le manchon d'adaptateur (1) est suffisamment grande.

10. Emballage selon la revendication 9, **caractérisé par le fait que** les moyens sont des plaquettes (12) qui sont séparées l'une de l'autre par un écarteur.

11. Emballage selon la revendication 8 ou 9, **caractérisé par le fait que**, en cas de contact, les matières ou le revêtement des moyens ou des plaquettes (12) provoquent une réaction chimique avec changement de couleur.

12. Emballage selon une des revendications précédentes, **caractérisé par le fait qu'**un élément piézoélectrique (15), qui mesure et indique la force d'emmanchement, est disposé le cas échéant, en plus du disque anti-usure d'emballage (14), entre le manchon d'adaptateur (1) et l'emballage (3), l'élément de signalisation pouvant par exemple être une lampe (17) ou une source sonore.

13. Emballage selon une des revendications précédentes, **caractérisé par le fait que** la saillie est conformée en téton (7) conique, sur lequel un disque anti-usure d'emballage (14) est disposé de manière à pouvoir être déplacé dans le sens longitudinal du téton (7) en cas d'application d'une pression, et le manchon d'adaptateur (1) repose sur le disque anti-usure d'emballage (14), en entourant le téton (7), et déplace le disque anti-usure d'emballage (14) lorsque la tête sphérique (4) est pressée sur le manchon d'adaptateur (1).

14. Emballage selon une des revendications précédentes, **caractérisé par le fait que** l'endroit jusqu'auquel le disque anti-usure d'emballage (14) doit être déplacé en cas d'application d'une pression sur le manchon d'adaptateur (1) par la tête sphérique (4) est marqué ou est une butée, par exemple le fond de l'emballage.

15. Emballage selon une des revendications précédentes, **caractérisé par le fait qu'**une tête sphérique (4) est en même temps positionnée de façon détachable dans l'emballage (3) et que la tête sphérique peut être retirée séparément.
